# EUROPEAN PATENT SPECIFICATION

(11) **EP 2 221 036 B1**
(45) Date of publication and mention of the grant of the patent: **07.01.2015**
(21) Application number: 08733914.9
(22) Date of filing: 07.04.2008
(51) Int. Cl.: A61G 11/00

(54) **An infant incubator with radiant heater**
Inkubator für Säuglinge mit Heizstrahler
Couveuse à bébés à chauffage rayonnant

(30) Priority: 18.01.2008 CN 200820082636 U
(43) Date of publication of application: 25.08.2010
(73) Proprietor: Ningbo David Medical Device Co., Ltd., Ningbo Zhejiang Province 315712 (CN)
(72) Inventor: CHEN, Zaihong, Ningbo Zhejiang Province 315712 (CN)
(74) Representative: Kling, Simone
(86) International application number: PCT/CN2008/000701
(87) International publication number: WO 2009/089666

(56) References cited:
- EP-A2- 1 053 736
- CN-A- 1 929 804
- CN-Y- 200 948 212
- CN-Y- 201 036 600
- US-A- 3 158 150
- US-A- 5 759 149
- US-B2- 6 669 625

## Description

### FIELD OF THE INVENTION

The present invention relates to an urgent treatment device for a newborn infant in delivery room, in particular to an infant incubator with radiant heater which can maintain a humid and warm atmosphere and thus provide a good rescue disposal environment for infant.

### BACKGROUND OF THE INVENTION

An ideal infant incubator with radiant heater can not only perform functions of heating and humidifying but also maintain temperature and humidity to ensure that the temperature and humidity inside the infant incubator with radiant heater are in an ideal state. Therefore, it can ensure that there is a good life-support environment for an infant in the infant incubator with radiant heater.

However, conventional infant incubator with radiant heater includes an infrared radiant source disposed above an infant bed. The heat balance of an infant patient is maintained by the direct radiation of the infrared spectrum. Such thermal radiation only acts on the radiated side of the infant patient, while the back of the infant patient is still cool. Thus, such infant incubator is far from perfect because the temperature and humidity in the infant bed are not uniform enough. Besides, as a rescue platform for infant patients, conventional infant incubator with radiant heater cannot form a complete, stable and long--lasting maintenance for warm atmosphere because it is fully open. The direct thermal radiation of the infant incubator with radiant heater easily causes dehydration for the infant patient, and thus it is possible to bring about some unfavorable conditions to the infant patient.

In addition, the bed of the conventional infant incubator with radiant heater is inclined and adjusted manually. Such manual adjustment tends to generate undesired vibration and cannot determine the inclined angle, which are disadvantage for the normal nursing for the infant patient. US 5,759,149 describes a patient support system having an environmentally controlled area over the patient including a frame with a patient support thereon and a movable canopy thereover.

### SUMMARY OF THE INVENTION

In view of the disadvantages in the prior art, the technical problem to be solved by the present invention is to provide an infant incubator with radiant heater, which allows temperature and humidity in the infant bed to be relatively uniform to facilitate the formation of a complete, stable and long-lasting maintenance for humid and warm air, thereby ensuring a good life-support environment for an infant.

In order to solve the above technical problem, the present invention provides an infant incubator with radiant heater comprising a radiant means with an infrared heater, wherein an infant bed is provided below the radiant means and comprises a humidifier and a second thermostat, **characterized in that** a transparent sphere cover is mounted around the infant bed, a volute casing having a circulating blower and a cover board for directing air are provided under the infant bed, the cover board includes an air outlet and an air return inlet, humid and warm air generated by the second thermostat and the humidifier flows through the circulating blower in the volute casing, the air outlet in the cover board, and then is directed by a cambered surface of the transparent sphere cover to a surface of the infant bed, finally, enters the air return inlet so as to achieve a circulation of humid and warm air, thereby forming an appropriate humid and warm environment around the infant bed.

With the above configuration, the temperate and humidity in the infant bed may be relatively uniform and the heating for the transparent sphere cover is always controlled in a controllable and stable range to facilitate the formation of a complete, stable and long-lasting maintenance for humid and warm air, thereby ensuring a good nursing environment for infants in the infant incubator with radiant heater.

The air outlet in the cover board is an outlet means for discharging the humid and warm air in a 360 degree range, which helps the humid and warm air to uniformly reach the surface of the bed for the infant patient.

The transparent sphere cover can be opened toward three sides, and when the transparent sphere cover is opened, air is still expelled from the air outlet in the cover board and forms an air curtain, which can isolate the inside of the transparent sphere cover from external temperature and allow the environment in the incubator always in a stable state..

Another technical problem to be solved by the present invention is to adjust inclination of the infant bed without vibration and to operate more simply. For this purpose, an inclining means, a lift mechanism and a lower cabinet are connected below the infant bed with the transparent sphere cover, and the lower cabinet is provided with a power box with a door, a lift switch, a drawer and casters. The inclining means may avoid vibration caused by manual adjustment and may exactly identify the inclination angle. The door of the power box may be opened to facilitate the connection with power, and may be closed to protect the connector when the plug is detached from the socket. Due to the provision of the lower cabinet, the internal elements and devices inside the lower cabinet may be enveloped, the drawer may be easily pulled out and pushed in, and the infant incubator may have an attractive appearance.

The radiant means may comprise a radiant cover having a upper parabolic reflector plate and a lower reflector plate, the infrared heater may be provided at the upper parabolic reflector plate, a blue light lamp may be provided at the lower reflector plate, an illuminating means may be disposed at the upper portion of the radiant cover, and a transparent cover may be disposed at the lower portion of the radiant cover. The second thermostat may regulate the power of the heater in the radiant means, and may control the blue light lamp, the illuminating means and the humidifier in the upper cabinet.

Preferably, a stand is provided below the radiant means, and a lower end of the stand is connected to a support mechanism below the transparent sphere cover. The radiant means is rotatable relative to the stand within a range of more than ±90 degrees in a horizontal surface.

The infrared heating means may be consisted of the oval-type infrared heater and the upper parabolic reflector plate. The radiant means may regulate the intensity of the infrared heater by the first thermostat. The radiant means has a radiating area larger than the bed for the infant patient, and uniformly radiates the infant patient with infrared light in an oval shape, which may radiate the infant patient with infrared light more uniformly and efficiently.

Compared with the prior art, because of the transparent sphere cover, the volute casing with the circulating blower and the cover board for directing air with an air outlet and air return inlets which are disposed below the infant bed, the present invention has the advantage that the temperate and humidity in the infant bed are relatively uniform to facilitate formation of a complete, stable and long-lasting maintenance for humid and warm air, thereby ensuring a good life-support environment for the infant in the infant incubator with radiant heater.

### BRIEF DESCRIPTION OF THE DRAWINGS

Fig. 1 is a schematic structural view of the present invention ;
Fig. 2 is a view seeing along "B" direction in Fig. 1 ; and
Fig. 3 is a schematic view of a system for maintaining humid and warm air in the bed-sphere cover assembly according to the present invention.

### DETAILED DESCRIPTION OF THE INVENTION

Hereinafter, the present invention will be further described in detail with reference to the embodiment shown in the drawings.

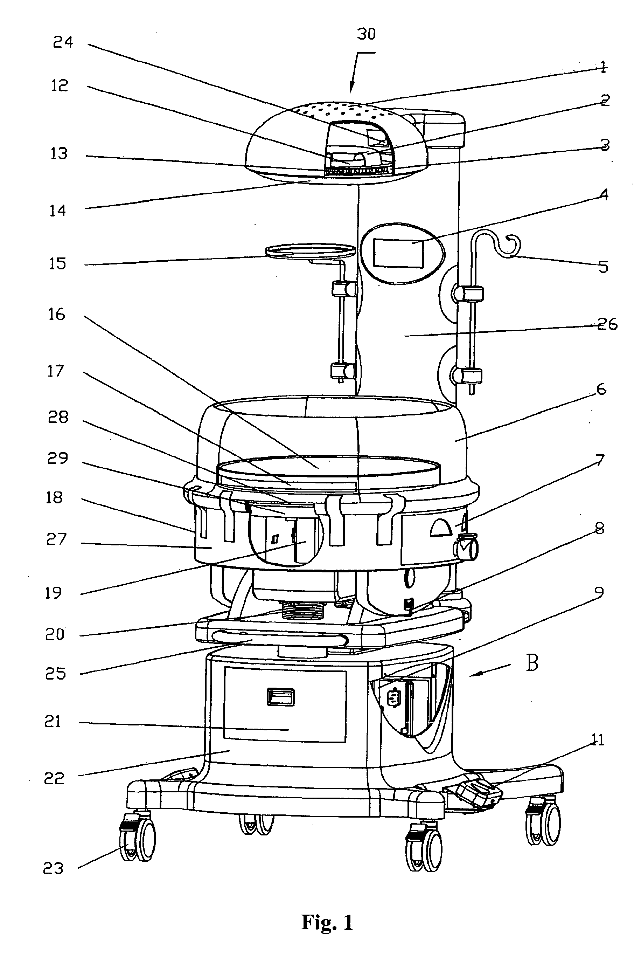

An embodiment of an infant incubator with radiant heater according to the present invention is shown in Figs. 1 to 3.

**Reference numeral in the Figures:**

| | | | |
|---|---|---|---|
| 1- | radiant cover, | 2- | upper parabolic reflector plate, |
| 3- | lower reflector plate, | 4- | first thermostat, |
| 5- | transfusion support, | 6- | transparent sphere cover, |
| 7- | humidifier, | 8- | inclination indicator, |
| 9- | lift mechanism, | 10- | power box with a door, |
| 11- | lift switch, | 12- | infrared heater, |
| 13- | blue light lamp, | 14- | transparent cover, |
| 15- | tray, | 16- | infant bed, |
| 17- | radiographing box, | 18- | second thermostat, |
| 19- | volute casing, | 20- | inclining means, |
| 21- | drawer, | 22- | lower cabinet, |
| 23- | casters, | 24- | illuminating means, |
| 25- | handle, | 26- | stand, |
| 27- | upper cabinet, | 28- | cover board, |
| 29- | guide baffle. | | |

As shown in Fig. 1, the infant incubator with radiant heater according to the present invention includes a radiant means 30 installed at the upper end of a stand 26, a bed-sphere cover assembly installed at the lower end of the stand 26 and including a support mechanism, a inclining means 20 installed below the bed-sphere cover assembly, a lift mechanism 9, a lower cabinet 22 and a power box with a door 10.

The radiant means includes a radiant cover 1 having an upper parabolic reflector plate 2 and a lower reflector plate 3. An infrared heater 12 is provided at the upper parabolic reflector plate, and a blue light lamp 13 is provided at the lower reflector plate 3. An illuminating means 24 is disposed at the upper portion of the radiant cover 1, and a transparent cover 14 is disposed at the lower portion of the radiant cover 1. The radiant means is rotatable relative to the stand 26 within a range of more than ±90 degrees in a horizontal surface. The intensity of the infrared heater 12 of the radiant means is controlled by a first thermostat 4. The infrared heater 12 has a radiating area larger than the bed for an infant patient, and radiates the infant patient uniformly in an oval shape.

A heating means consisted of the oval-type infrared heater 12 and the upper parabolic reflector plate 2 may radiate the infant patient with light more uniformly and efficiently.

The first thermostat 4, a transfusion support 5 and a tray 15 are disposed on the stand 26.

When being boxed up, after a support plate in the support mechanism is detached, the radiant means, together with the stand, may be placed above a transparent sphere cover 6 of the bed-sphere cover assembly with a certain distance therebetween, which facilitates the transportation and the installation. A handle 25 disposed on the support mechanism facilitates the motion of the infant incubator with radiant heater according to the present invention.

The bed-sphere cover assembly includes an infant bed 16 above which the transparent sphere cover 6 is disposed. An upper cabinet 27 is connected to the lower end of the infant bed. The upper cabinet 27 is provided with a radiographing box 17, a second thermostat 18, a humidifier 7 and a volute casing 19.

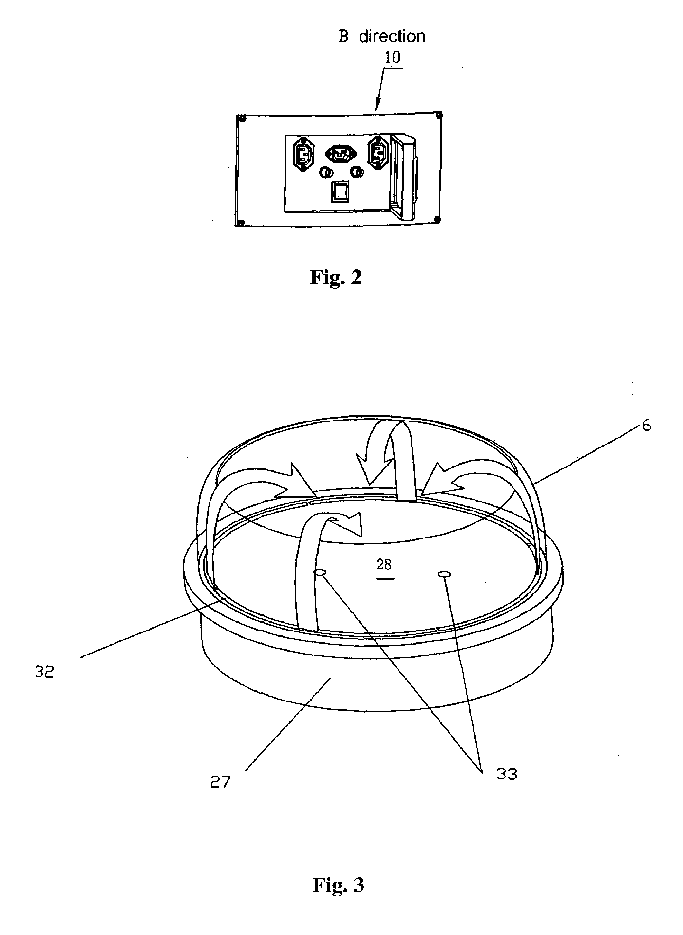

A system for maintaining humid and warm air in the bed-sphere cover assembly according to the present invention may operate as follows. As shown in Fig. 3, humid and warm air generated by the second thermostat and the humidifier flows through the circulating blower within the volute casing 19, the air outlet in the cover board 28, and then is directed by the cambered surface of the transparent sphere cover 6 to the infant bed 16, finally, enters a humid and warm air return inlet 33 in the cover board so as to achieve a circulation of humid and warm air.

The lower cabinet 22 is provided with a power box with a door 10 (as shown in Fig. 2), a lift switch 11, a drawer 21 and casters 23. The door of the power box may be opened to facilitate the connection with power, and may be closed to protect the connector when the plug is detached from the socket. Due to the provision of the lower cabinet, the internal elements and devices inside the lower cabinet may be enveloped, the drawer may be easily pulled out and pushed in, and the infant incubator may have an attractive appearance.

The second thermostat 18 may regulate the power of the heater in the radiant means, and may control the blue light lamp, the illuminating means and the humidifier in the upper cabinet.

The inclining means 20 may regulate the inclination angle of the bed-sphere cover assembly within a range of more than ±12 degree. The first thermostat 4 may display and provide an alarm by a rate timer, a bed-sphere cover assembly temperature sensor, a skin temperature sensor and a duct sensor. The heating means may regulate the temperature in the incubator depending on the change of ambient temperature, such that the temperature of the bed-sphere cover assembly is always controlled in a relative stable range.

In the infant incubator with radiant heater according to the present invention, an inclining mechanism consisted of the inclining means, the bed-sphere cover assembly and the inclination indicator may avoid vibration and may identify the inclination angle so as to facilitate the appropriate nursing for infant patients.

In the infant incubator with radiant heater according to the present invention, a humid and warm air generated by the second thermostat and the humidifier within the upper cabinet 27 flows through the circulating blower within the volute casing 19, the air outlet 32 in the cover board 28, and then is directed by the cambered surface of the transparent sphere cover 6 to the bed 16, finally, enters the humid and warm air return inlet 33 in the cover board 28 so as to achieve a circulation of humid and warm air, thereby achieving an appropriate humid and warm environment around the infant bed.

The inclining mechanism of the present invention, which is consisted of the inclining means 20, the bed-sphere cover assembly and the inclination indicator, may be electrically controlled so as to avoid vibration caused by manual adjustment and may exactly identify the inclination angle.

The present invention has an outlet means for discharging the humid and warm air in a 360 degree range, which is an air outlet 32 having a certain width and disposed in the cover board below the bed along the periphery of the transparent sphere cover, so as to help the uniform humid and warm air to reach the surface of the bed for infant patient.

Due to the provision of the lower cabinet, the internal elements and devices inside the lower cabinet may be enveloped, the drawer may be easily pulled out and pushed in, and the infant incubator may have an attractive appearance.

The transparent sphere cover 6 of the present invention may be opened towards three directions or three sides. When the transparent sphere cover 6 is opened, air is still expelled from the air outlet and forms an air curtain, which can isolate the inside of the transparent sphere cover 6 from external temperature and allow the environment in the incubator always in a stable state.

In the present invention, the air outlet of the volute casing 19 is positioned in the center of the guide baffle 29 and the air return inlets are located in the left and right sides of the cover board 28 such that temperature and humidity within the bed-sphere cover assembly may be relatively uniform.

## Claims

1. An infant incubator with radiant heater comprising a radiant means (30) with an infrared heater (12), wherein an infant bed (16) is provided below the radiant means (30) and comprises a humidifier (7) and a second thermostat (18), **characterized in that**
a transparent sphere cover (6) is mounted around the infant bed (16), a volute casing (19) having a circulating blower and a cover board (28) for directing air are provided under the infant bed (16), the cover board includes an air outlet (32) and an air return inlet (33), humid and warm air generated by the second thermostat (18) and the humidifier (7) flows through the circulating blower in the volute casing (19), the air outlet (32) in the cover board, and then is directed by a cambered surface of the transparent sphere cover (6) to a surface of the infant bed (16), finally, enters the air return inlet (33) so as to achieve a circulation of humid and warm air, thereby forming an appropriate humid and warm environment around the infant bed (16).

2. The infant incubator with radiant heater according to claim 1, wherein the air outlet (32) in the cover board is an outlet means for discharging the humid and warm air in a 360 degree range.

3. The infant incubator with radiant heater according to claim 1, wherein the transparent sphere cover (6) can be opened toward three sides, and when the transparent sphere cover (6) is opened, air is still expelled from the air outlet (32) in the cover board and forms an air curtain.

4. The infant incubator with radiant heater according to claim 1, wherein the radiant means (30) comprises a radiant cover (1) having a upper parabolic reflector plate (2) and a lower reflector plate (3), the infrared heater (12) is provided at the upper parabolic reflector plate (2), a blue light lamp (13) is provided at the lower reflector plate (3), an illuminating means (24) is disposed at the upper portion of the radiant cover (1), and a transparent cover (14) is disposed at the lower portion of the radiant cover (1).

5. The infant incubator with radiant heater according to claim 4, wherein a stand (26) is provided below the radiant means (30), and a lower end of the stand is connected to a support mechanism below the transparent sphere cover (6).

6. The infant incubator with radiant heater according to claim 5, wherein the radiant means (30) is rotatable relative to the stand (26) within a range of more than ±90 degrees in a horizontal surface.

7. The infant incubator with radiant heater according to claim 1, wherein a lower cabinet (22) is disposed below the infant bed (16) and comprises an power box with a door (10), a lift switch (11), a drawer (21) and a plurality of casters (23).

## Patentansprüche

1. Inkubator für Säuglinge mit Heizstrahler, der ein Strahlungsmittel (30) mit einem Infrarotstrahler (12) umfasst, wobei ein Säuglingsbett (16) unterhalb des Strahlungsmittels (30) vorgesehen ist und einen Befeuchter (7) und ein zweites Thermostat (18) umfasst, **dadurch gekennzeichnet, dass**
eine transparente Kugelabdeckung (6) um das Säuglingsbett (16) herum angebracht ist, ein Spiralgehäuse (19) mit einem Umluftgebläse und ein Abdeckbrett (28) zum Leiten von Luft unter dem Säuglingsbett (16) vorgesehen sind, das Abdeckbrett einen Luftauslass (32) und einen Luftrückführungseinlass (33) enthält, feuchte und warme Luft, die vom zweiten Thermostat (18) und dem Befeuchter (7) erzeugt wird, durch das Umluftgebläse im Spiralgehäuse (19) und den Luftauslass (32) im Abdeckbrett strömt und danach von einer gewölbten Fläche der transparenten Kugelabdeckung (6) auf eine Fläche des Säuglingsbetts (16) geleitet wird, schließlich in den Luftrückführungseinlass (33) eintritt, um eine Umluft feuchter und warmer Luft zu erzielen, wodurch eine geeignete feuchte und warme Umgebung um das Säuglingsbett (16) herum gebildet wird.

2. Inkubator für Säuglinge mit Heizstrahler nach Anspruch 1, wobei der Luftauslass (32) im Abdeckbrett ein Ausgabemittel zum Ausgeben der feuchten und warmen Luft in einem Bereich von 360 Grad ist.

3. Inkubator für Säuglinge mit Heizstrahler nach Anspruch 1, wobei die transparente Kugelabdeckung (6) zu drei Seiten hin geöffnet werden kann und wenn die transparente Kugelabdeckung (6) geöffnet ist, Luft immer noch aus dem Luftauslass (32) im Abdeckbrett ausgestoßen wird und einen Luftschleier bildet.

4. Inkubator für Säuglinge mit Heizstrahler nach Anspruch 1, wobei das Strahlungsmittel (30) eine Strahlungsabdeckung (1) mit einer oberen Parabolreflektorplatte (2) und einer unteren Parabolreflektorplatte (3) umfasst, wobei der Infrarotstrahler (12) an der oberen Parabolreflektorplatte (2) angebracht ist, eine Blaulichtlampe (13) an der unteren Reflektorplatte (3) vorgesehen ist, ein Beleuchtungsmittel (24) am oberen Abschnitt der Strahlungsabdeckung (1) angeordnet ist und eine transparente Abdeckung (14) am unteren Abschnitt der Strahlungsabdeckung (1) angeordnet ist.

5. Inkubator für Säuglinge mit Heizstrahler nach Anspruch 4, wobei ein Gestell (26) unterhalb des Strahlungsmittels (30) vorgesehen ist und ein unteres Ende des Gestells mit einem Trägermechanismus unterhalb der transparenten Kugelabdeckung (6) verbunden ist.

6. Inkubator für Säuglinge mit Heizstrahler nach Anspruch 5, wobei das Strahlungsmittel (30) in Bezug auf das Gestell (26) innerhalb eines Bereichs von mehr als ± 90 Grad in einer horizontalen Fläche drehbar ist.

7. Inkubator für Säuglinge mit Heizstrahler nach Anspruch 1, wobei ein unterer Schrank (22) unterhalb des Säuglingsbetts (16) angeordnet ist und einen Netzanschlusskasten mit einer Tür (10), einem Hebeschalter (11), einer Schublade (21) und einer Mehrzahl von Laufrollen (23) umfasst.

## Revendications

1. Couveuse pour bébé avec chauffage rayonnant comprenant un moyen rayonnant (30) avec un dispositif de chauffage à infrarouge (12), dans lequel un lit de bébé (16) est prévu au-dessous du moyen rayonnant (30) et comprend un humidificateur (7) et un second thermostat (18), **caractérisée en ce que** :
un couvercle sphérique transparent (6) est monté autour du lit de bébé (16), une volute (19) ayant un ventilateur circulant et une plaque de couvercle (28) pour diriger l'air, sont prévus sous le lit de bébé (16), la plaque de couvercle comprend une sortie d'air (32) et une entrée de refoulement d'air (33), l'air humide et chaud généré par le second thermostat (18) et l'humidificateur (7) s'écoule à travers le ventilateur circulant dans la volute (19), la sortie d'air (32) dans la plaque de couvercle, et est ensuite dirigé par la surface courbée du couvercle sphérique transparent (6) jusqu'à une surface du lit de bébé (16), finalement, entre dans l'entrée de refoulement d'air (33) afin d'obtenir une circulation d'air humide et chaud, formant ainsi un environnement humide et chaud approprié autour du lit de bébé (16).

2. Couveuse pour bébé avec chauffage rayonnant selon la revendication 1, dans laquelle la sortie d'air (32) dans la plaque de couvercle est un moyen de sortie pour décharger l'air humide et chaud dans une plage de 360 degrés.

3. Couveuse pour bébé avec chauffage rayonnant selon la revendication 1, dans laquelle le couvercle sphérique transparent (6) peut être ouvert vers trois côtés, et lorsque le couvercle sphérique transparent (6) est ouvert, l'air est encore expulsé par la sortie d'air (32) dans la plaque de couvercle et forme un rideau d'air.

4. Couveuse pour bébé avec chauffage rayonnant selon la revendication 1, dans laquelle le moyen rayonnant (30) comprend un couvercle rayonnant (1) ayant une plaque réfléchissante parabolique supérieure (2) et une plaque réfléchissante inférieure (3), le dispositif de chauffage à infrarouge (12) est prévu au niveau de la plaque réfléchissante parabolique supérieure (2), une lampe de lumière bleue (13) est prévue au niveau de la plaque réfléchissante inférieure (3), un moyen d'éclairage (24) est disposé au niveau de la partie supérieure du couvercle rayonnant (1), et un couvercle transparent (14) est disposé au niveau de la partie inférieure du couvercle rayonnant (1).

5. Couveuse pour bébé avec chauffage rayonnant selon la revendication 4, dans laquelle un support (26) est prévu au-dessous des moyens rayonnants (30), et une extrémité inférieure du support est raccordée à un mécanisme de support au-dessous du couvercle sphérique transparent (6).

6. Couveuse pour bébé avec chauffage rayonnant selon la revendication 5, dans laquelle le moyen rayonnant (30) peut tourner par rapport au support (26) dans une plage supérieure à ±90 degrés dans une surface horizontale.

7. Couveuse pour bébé avec chauffage rayonnant selon la revendication 1, dans laquelle une armoire inférieure (22) est disposée sous le lit de bébé (16) et comprend un boîtier d'alimentation avec une porte (10), un interrupteur de levage (11), un tiroir (21) et une pluralité de roulettes (23).
